(19) Europäisches Patentamt
European Patent Office
Office européen des brevets

(11) Veröffentlichungsnummer : **0 581 738 A1**

(12) # EUROPÄISCHE PATENTANMELDUNG

(21) Anmeldenummer : **93810525.1**

(51) Int. Cl.[5] : **C07D 401/12,** A01N 47/36

(22) Anmeldetag : **21.07.93**

(30) Priorität : **30.07.92 CH 2402/92**
**02.02.93 CH 313/93**

(43) Veröffentlichungstag der Anmeldung :
**02.02.94 Patentblatt 94/05**

(84) Benannte Vertragsstaaten :
**AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Föry, Werner, Dr.**
**Inzlingerstrasse 11**
**CH-4125 Riehen (CH)**
Erfinder : **Schurter, Rolf, Dr.**
**Holzmattstrasse 45**
**CH-4102 Binningen (CH)**

(54) **Herbizide Pyridylsulfonylharnstoffe.**

(57) (Cyclopropyl-triazinyl- und Cyclopropyl-pyrimidinyl)-pyridylsulfonylharnstoffe der Formel I

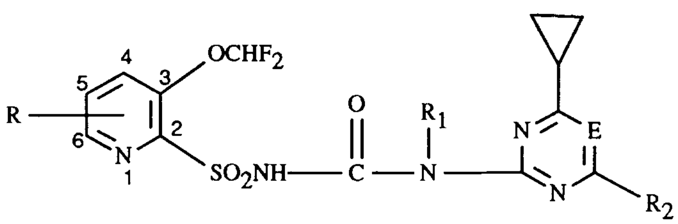

(I),

worin
R und $R_1$ unabhängig voneinander Wasserstoff oder Methyl sind ;
$R_2$ Methyl, Methoxy oder Ethoxy ; und
E Stickstoff oder die Methingruppe bedeutet, sowie die Salze dieser Verbindungen haben gute selektiv-herbizide Eigenschaften. Die Herstellung dieser Verbindungen und ihre Verwendung als herbizide Wirkstoffe sind beschrieben.

EP 0 581 738 A1

Die vorliegende Erfindung betrifft neue, herbizid wirksame (Cyclopropyl-triazinyl- und Cyclopropyl-pyrimidinyl)-pyridylsulfonylharnstoffe, Verfahren zu ihrer Herstellung, Mittel, die diese (Cyclopropyl-triazinyl- und Cyclopropyl-pyrimidinyl)-pyridylsulfonylharnstoffe als Wirkstoffe enthalten, sowie ihre Verwendung zum Bekämpfen von Unkräutern, vor allem in Nutzpflanzenkulturen wie z.B. Getreide, Baumwolle, Soja, Mais, Reis und bevorzugt Raps.

N-Pyridinylsulfonyl-N'-pyrimidinyl- und -triazinylharnstoffe mit herbizider Wirkung sind bereits bekannt und beispielsweise in EP-A-0 103 543 und US-A-4 544 401 beschrieben.

Es wurden nun neue Sulfonylharnstoffe mit herbiziden Eigenschaften gefunden.

Die erfindungsgemässen (Cyclopropyl-triazinyl- und Cyclopropyl-pyrimidinyl)-pyridylsulfonylharnstoffe entsprechen der Formel I

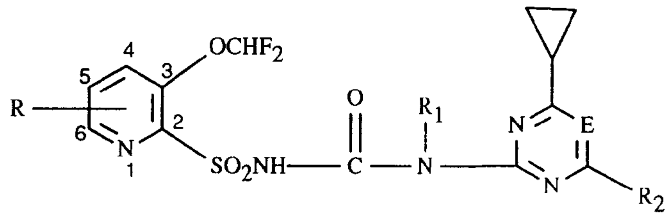
(I),

worin

R und $R_1$ unabhängig voneinander Wasserstoff oder Methyl sind;

$R_2$ Methyl, Methoxy oder Ethoxy; und

E Stickstoff oder die Methingruppe bedeutet, sowie die agrochemisch verträglichen Salze dieser Verbindungen.

Die Erfindung umfasst auch die Salze, die die Verbindungen der Formel I als Sulfonamide mit saurem Proton mit Aminen, Alkali- und Erdalkalimetallbasen oder quaternären Ammoniumbasen bilden können.

Beispiele für zur Salzbildung geeignete Amine sind primäre, sekundäre und tertiäre aliphatische und aromatische Amine wie Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-isopropylamin, Methyl-hexylamin, Methyl-nonylamin, Methyl-pentadecylamin, Methyl-octadecylamin, Ethyl-butylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Diheptylamin, Dioctylamin, Ethanolamin, n-Propanolamin, iso-Propanolamin, N,N-Diethylethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Diethanolamin, Trimethylamin, Triethylamin, Tri-n-propylamin, Tri-iso-propylamin, Tri-n-butylamin, Tri-iso-butylamin, Tri-sek.-butylamin, Tri-n-amylamin; heterocyclische Amine wie z.B. Pyridin, Chinolin, iso-Chinolin, Morpholin, N-Methylmorpholin, Thiomorpholin, Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline; insbesondere aber Ethyl-, Propyl-, Diethyl- oder Triethylamin, vor allem aber iso-Propylamin und Diethanolamin.

Beispiele für quaternäre Ammoniumbasen sind im allgemeinen die Kationen vonHalogenammoniumsalzen, z.B. das Tetramethylammoniumkation, das Trimethylbenzylammoniumkation, das Triethylbenzylammoniumkation, das Tetraethylammoniumkation, das Trimethylethylammoniumkation, aber auch das Ammoniumkation.

Unter Alkali- und Erdalkalimetallbasen als Salzbildner sind die Hydroxyde von Lithium, Magnesium oder Calcium, insbesondere von Natrium oder Kalium hervorzuheben.

Bevorzugt sind die Verbindungen der Formel I, worin E die Methingruppe bedeutet.

Bevorzugt sind ebenfalls die Verbindungen der Formel $I_a$

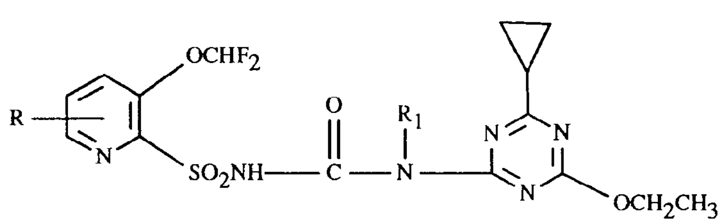
($I_a$),

worin R und $R_1$ die oben angegebenen Bedeutungen haben.

Ferner sind bevorzugt Verbindungen der Formel I, worin R in 6-Position gebunden ist.

Besonders bevorzugt ist die Verbindung der Formel $I_b$

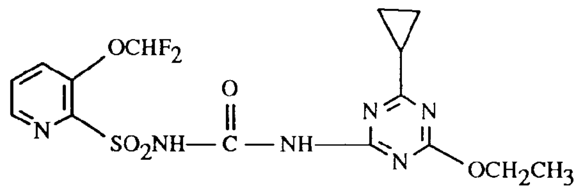

($I_b$).

Die Verbindungen der Formel I können hergestellt werden, indem man entweder

a) ein 2-Pyridylsulfonamid der Formel II

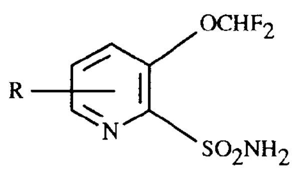

(II),

worin R die angegebene Bedeutung hat, mit einem N-2-Pyrimidinyl- oder N-2-Triazinylcarbamat der Formel III

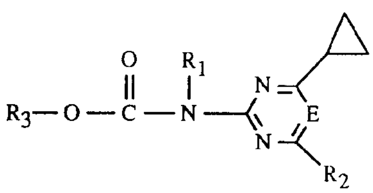

(III),

worin $R_1$, $R_2$ und E die unter Formel I angegebenen Bedeutungen haben und $R_3$ Phenyl oder 4-Tolyl ist, in Gegenwart einer Base umsetzt, oder

b) ein 2-Pyridylsulfonamid der Formel IV

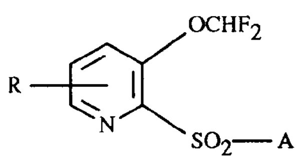

(IV),

worin R die angegebene Bedeutung hat, und A

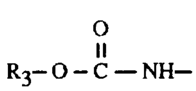

oder O=C=N- ist, wobei $R_3$ die oben angegebene Bedeutung hat, gegebenenfalls in Gegenwart einer Base mit einem 2-Aminopyrimidin- oder -triazin der Formel V

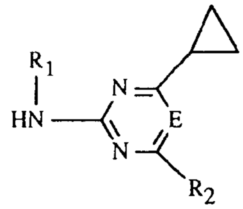

(V),

worin $R_1$, $R_2$ und E die angegebenen Bedeutungen haben, umsetzt, oder

c) ein 2-Pyridylsulfonylchlorid der Formel VI

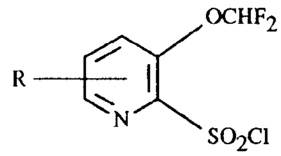

(VI),

worin R die angegebene Bedeutung hat, mit einem Metall-Cyanat der Formel VII

$$O{=}C{-}N^{\ominus}M^{\oplus} \quad (VII),$$

worin $M^{\oplus}$ ein Ammonium-, Phosphonium-, Sulfonium- oder ein Alkalimetall-Kation ist, und einem 2-Aminopyrimidin- oder -triazin der Formel Va

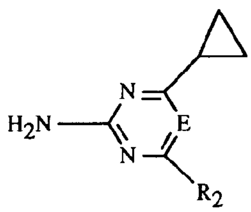

(Va),

worin $R_2$ und E die angegebenen Bedeutungen haben, gegebenenfalls in Gegenwart einer Base in einem inerten, organischen Lösungsmittel, umsetzt, oder

d) ein 2-Pyridylsulfonamid der Formel II

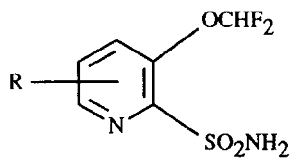

(II),

worin R die angegebene Bedeutung hat, gegebenenfalls in Gegenwart einer Base mit einem 2-Pyrimidinyl- oder 2-Triazinylisocyanat der Formel VIII

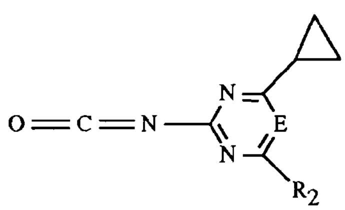

(VIII),

worin $R_2$ und E die angegebenen Bedeutungen haben, umsetzt.

Die Verfahrensvarianten a), b), c) und d) folgen dem Reaktionsschema 1.

Reaktionsschema 1:

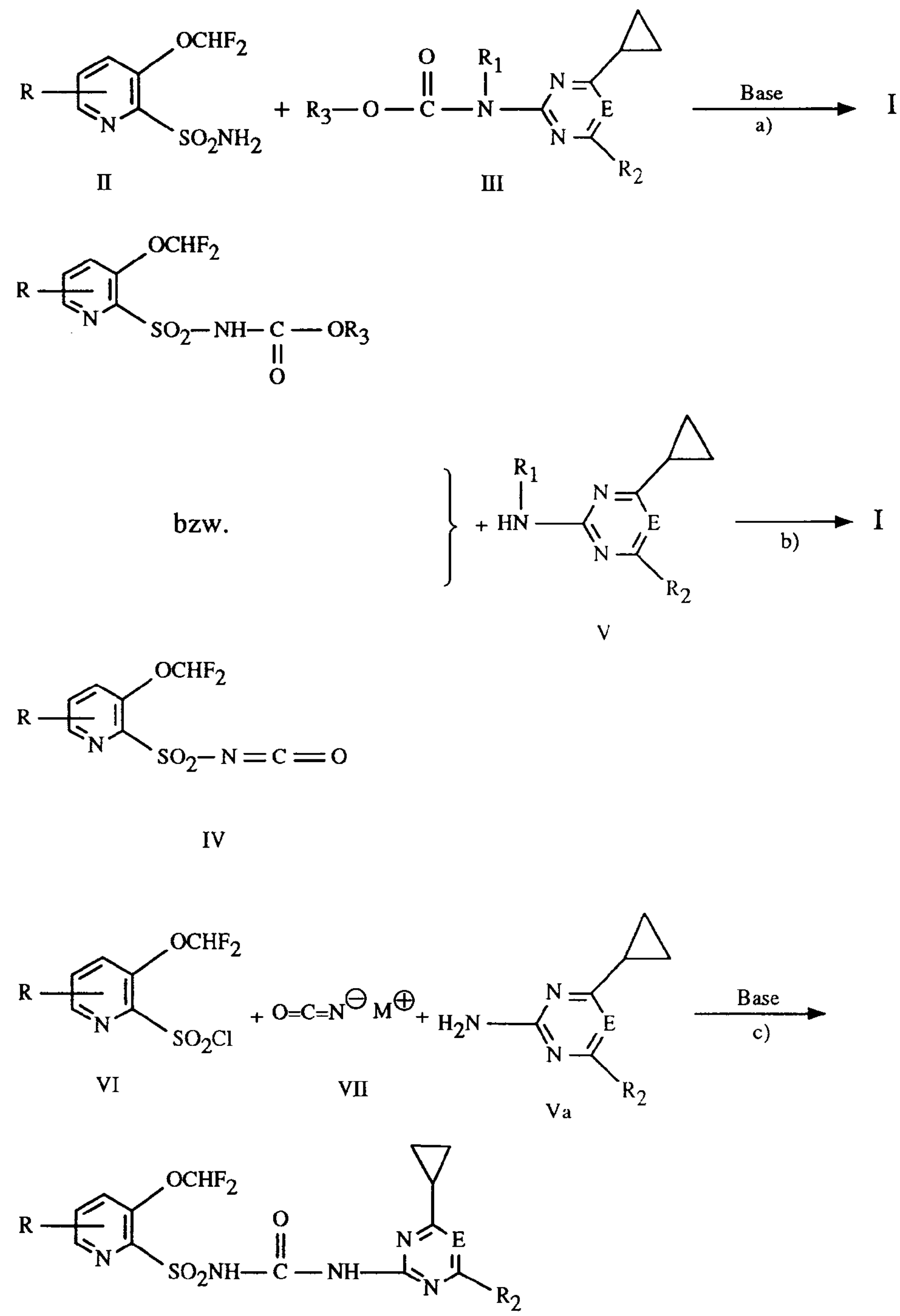


II III I

bzw.

IV V I

VI VII Va

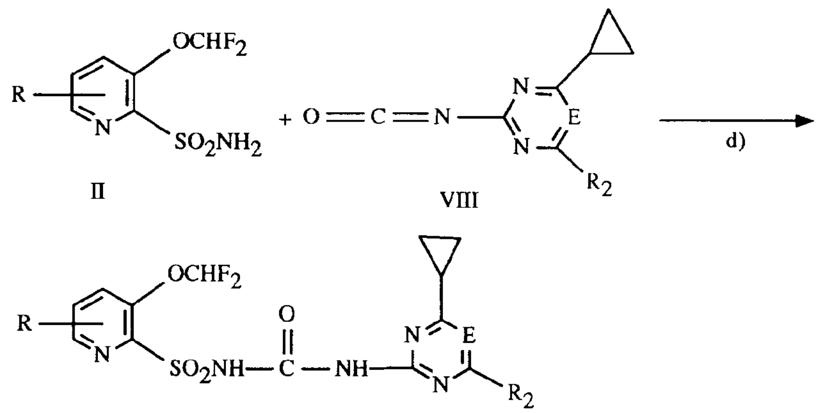


II VIII

Die Umsetzungen zu Verbindungen der Formel I nach Verfahrensvarianten a) bis d) werden zweckmässigerweise in aprotischen, inerten, organischen Lösungsmitteln durchgeführt. Solche Lösungsmittel sind Kohlenwasserstoffe wie Benzol, Toluol, Xylol, Hexan oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie Dimethylformamid, Diethylformamid oder N-Methylpyrrolidon, und Ester wie Essigsäureethylester. Die Reaktionstemperaturen liegen vorzugsweise zwischen 0°C und +150°C.
Die Umsetzungen verlaufen im allgemeinen ohne Exothermie oder nur leicht exothermisch und können zwischen +20°C und + 60°C durchgeführt werden. Zwecks Abkürzung der Reaktionszeit oder auch zum Einleiten der Umsetzung wird zweckdienlich für kurze Zeit zum Siedepunkt des Reaktionsgemisches aufgewärmt. Die Reaktionszeiten können ebenfalls durch Zugabe, entweder katalytischer Mengen, oder bis zu 2 Aequivalenten einer Base als Reaktionskatalysator verkürzt werden. Als Basen sind insbesondere tertiäre Amine wie Trimethylamin, Triethylamin, Chinuclidin, 1,4-Diazabicyclo-(2,2,2)-octan,1,5-Diazabicyclo(4,3,0)non-5-en oder 1,5-Diazabicyclo(5,4,0)undec-5-en oder Pyridin und 4-(N,N-Dimethylamino)-pyridin geeignet. Als Basen können aber auch anorganische Basen wie Hydride, z.B. Natrium- oder Calciumhydrid, Hydroxyde, z.B. Natrium- und Kaliumhydroxyd, Carbonate, z.B. Natrium- und Kaliumcarbonat oder Hydrogencarbonate, z.B. Kalium- und Natriumhydrogencarbonat, verwendet werden.

Die Endprodukte der Formel I können durch Einengen und/oder Verdampfen des Lösungsmittels isoliert und durch Umkristallisation oder Zerreiben des festen Rückstandes in Lösungsmitteln, in denen sie sich nicht gut lösen, beispielsweise verdünnte wässrige Säure wie 2N Salzsäure, Ether, Ester, aromatische Kohlenwasserstoffe oder chlorierte Kohlenwasserstoffe, gereinigt werden.
Eine weitere Möglichkeit für die Isolierung und Reindarstellung der Produkte der Formel I geht über (Flash-)Kieselgelchromatographie mit Hilfe eines geeigneten Lösungsmittels oder Lösungsmittelgemisches, z.B. Essigsäureethylester, Hexan oder Tetrahydrofuran.

Die für die Herstellungsverfahren benötigten Ausgangsverbindungen der Formel II, III, IV, V, Va, VI, VII und VIII sind entweder bekannt oder können aus bekannten Verbindungen analog zu bekannten Verfahren hergestellt werden. Verfahren zur Herstellung von 2-Pyridylsulfonamiden der Formel 11 sind beispielsweise in EP-A-0 103 543 beschrieben. Die Zwischenprodukte der Formel IV können aus den entsprechenden Sulfonamiden der Formel II analog zu bekannten Verfahren hergestellt werden. Derartige Umsetzungen sind z.B. in EP-A-0 103 543 beschrieben. Verfahren zur Herstellung der Zwischenprodukte der Formel III, V, Va und VIII sind beispielsweise in EP-A-0 108 708 beschrieben.

Die Verbindungen der Formel I können in unveränderter Form, d.h. wie sie in der Synthese anfallen, eingesetzt werden, vorzugsweise verarbeitet man sie aber auf übliche Weise mit den in der Formulierungstechnik üblichen Hilfsmitteln z.B. zu emulgierbaren Konzentraten, direkt versprühbaren oder verdünnbaren Lösungen, verdünnten Emulsionen, Spritzpulvern, löslichen Pulvern, Stäubemitteln, Granulaten oder Mikrokapseln. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit den Zusatzstoffen wie z.B. Lösungsmittel oder feste Trägerstoffe. Ferner können zusätzlich oberflächenaktive Verbindungen (Tenside) bei der Herstellung der Formulierungen verwendet werden.

Als Lösungsmittel können in Frage kommen: Aromatische Kohlenwasserstoffe, insbesondere die Fraktio-

nen $C_8$ bis $C_{12}$, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, stark polare Lösungsmittel wie N-Methyl-2-pyrrolidon, Dimethylsulfoxid oder Wasser; Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfahige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside, die auch in den erfindungsgemässen Mitteln verwendet werden können, sind u.a. in folgenden Publikationen beschrieben:

- "Mc Cutcheon's Detergents and Emulsifiers Annual", Mc Publishing Corp., Glen Rock, New Jersey, 1988.

- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die herbiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen: (% = Gewichtsprozent)

| <u>Emulgierbare Konzentrate:</u> | |
|---|---|
| Aktiver Wirkstoff: | 1 bis 90%, vorzugsweise 5 bis 50 % |
| oberflächenaktives Mittel: | 5 bis 30%, vorzugsweise 10 bis 20 % |
| Lösungsmittel: | 15 bis 94%, vorzugsweise 70 bis 85 % |

| <u>Stäube:</u> | |
|---|---|
| Aktiver Wirkstoff: | 0,1 bis 50 %, vorzugsweise 0,1 bis 5 % |
| festes Trägermittel: | 99,9 bis 90 %, vorzugsweise 99,9 bis 99 % |

| <u>Suspensions-Konzentrate:</u> | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

| <u>Benetzbare Pulver:</u> | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

| <u>Granulate:</u> | |
|---|---|
| Aktiver Wirkstoff: | 0,1 bis 30 %, vorzugsweise 0,1 bis 15 % |
| festes Trägermittel: | 99,5 bis 70 %, vorzugsweise 97 bis 85 % |

Die Wirkstoffe der Formel I werden in der Regel mit Aufwandmengen von 0,001 bis 2 kg/ha insbesondere 0,005 bis 1 kg/ha erfolgreich eingesetzt.

Die für die erwünschte Wirkung erforderliche Dosierung kann durch Versuche ermittelt werden. Sie ist abhängig von der Art der Wirkung, dem Entwicklungsstadium der Kulturpflanze und des Unkrauts, sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und

herbizide Eigenschaften aus, die sie ausgezeichnet zum Einsatz in Kulturen von Nutzpflanzen, insbesondere in Getreide, Baumwolle, Soja, Mais und Reis befähigen, wobei der Einsatz in Rapskultur ganz besonders bevorzugt ist.

Die nachfolgenden Beispiele erläutern die Erfindung weiter, ohne sie zu beschränken.

Herstellungsbeispiel:

Beispiel H1: Herstellung von N-(3-Difluormethoxypyridin-2-sulfonyl)-N'-(4-cyclopropyl-6-ethoxy-triazin-2-yl)-harnstoff (Verb.Nr. 1.001)

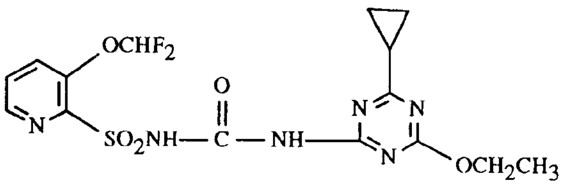

(1.001)

Zu einer Lösung von 2,24 g 3-Difluormethoxypyridin-2-yl-sulfonamid in 40 ml Acetonitril werden 1,63 ml 1,5-Diazabicyclo(5,4,0)undec-5-en, gefolgt von 3,15 g N-(4-cyclopropyl-6-ethoxy-triazin-2-yl)-phenylcarbamat gegeben. Die Reaktionsmischung wird während 45 Minuten bei Raumtemperatur gerührt und dann am Rotationsverdampfer eingeengt. Der ölige Rückstand wird mit 8 ml 2N Salzsäure verrieben und mit 10 ml Wasser verdünnt. Der Kristallbrei wird abfiltriert, mit Wasser und Diethylether gewaschen und getrocknet. Man erhält 4,05 g N-(3-Difluormethoxypyridin-2-sulfonyl)-N'-(4-cyclopropyl-6-ethoxy-triazin-2-yl)-harnstoff mit einem Schmelzpunkt von 144-145°C.

In analoger Weise werden die in der nachfolgenden Tabelle 1 aufgeführten Verbindungen der Formel I hergestellt.

Tabelle 1: Verbindungen der Formel I

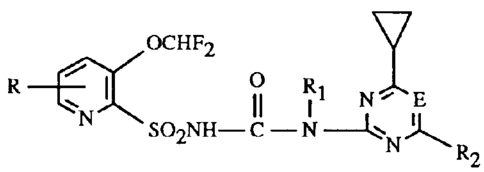

(I)

| Verb.Nr. | R | $R_1$ | $R_2$ | E | Phys-chem. Daten |
|---|---|---|---|---|---|
| 1.001 | H | H | $OC_2H_5$ | N | Smp. 144-145°C |
| 1.002 | 6-$CH_3$ | H | $OC_2H_5$ | N | Smp. 152-153°C (Zers.) |
| 1.003 | H | $CH_3$ | $OC_2H_5$ | N | |
| 1.004 | H | H | $OCH_3$ | N | Smp. 146-149°C |
| 1.005 | H | H | $OCH_3$ | CH | |
| 1.006 | H | H | $CH_3$ | CH | Smp. 137-139°C |
| 1.007 | 6-$CH_3$ | H | $CH_3$ | CH | |

Formulierungsbeispiele für Wirkstoffe der Formel I (% = Gewichtsprozent

| F1. Emulsionskonzentrate | a) | b) | c) | d) |
|---|---|---|---|---|
| Wirkstoff gemäß Tabelle 1 | 5 % | 10 % | 25 % | 50 % |
| Ca-Dodecylbenzolsulfonat | 6 % | 8 % | 6 % | 8 % |
| Ricinusöl-polyglykolether (36 Mol EO) | 4 % | - | 4 % | 4 % |
| Octylphenol-polyglykolether (7-8 Mol EO) | - | 4 % | - | 2 % |
| Cyclohexanon | - | - | 10 % | 20 % |
| Aromat. Kohlenwasserstoffgemisch $C_9$-$C_{12}$ | 85 % | 78 % | 55 % | 16 % |

Aus solchen Konzentraten können durch Verdünnung mit Wasser Emulsionen jeder gewünschten Konzentration hergestellt werden.

| F2. Lösungen | a) | b) | c) | d) |
|---|---|---|---|---|
| Wirkstoff gemäß Tabelle 1 | 5 % | 10 % | 50 % | 90 % |
| Dipropylenglykol-methylether | - | 20 % | 20 % | - |
| Polyethylenglykol MG 400 | 20 % | 10 % | - | - |
| N-Methyl-2-pyrrolidon | - | - | 30 % | 10 % |
| Aromat. Kohlenwasserstoffgemisch $C_9$-$C_{12}$ | 75 % | 60 % | - | - |

Die Lösungen sind zur Anwendung in Form kleinster Tropfen geeignet.

| F3. Spritzpulver | a) | b) | c) | d) |
|---|---|---|---|---|
| Wirkstoff gemäß Tabelle 1 | 1 % | 5 % | 25 % | 50 % |
| Na-Ligninsulfonat | 3 % | 4 % | - | 3 % |
| Na-Laurylsulfat | - | - | 3% | 1% |
| Na-Diisobutyl-naphtalinsulfonat | - | 3 % | 6 % | 5 % |
| Octylphenol-polyglykolether (7-8 Mol EO) | 2 % | 1 % | - | - |
| Hochdisperse Kieselsäure | 2 % | 2 % | 5 % | 5 % |
| Kaolin | 42 % | 35 % | 61 % | 36 % |
| Natriumchlorid | 50 % | 50 % | - | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F4. Umhüllungs-Granulate | a) | b) | c) |
|---|---|---|---|
| Wirkstoff gemäß Tabelle 1 | 0.1 % | 5 % | 15 % |
| Hochdisperse Kieselsäure | 0.9 % | 2 % | 2 % |
| Anorg. Trägermaterial (∅ 0.1 - 1 mm) wie z.B. $CaCO_3$ oder $SiO_2$ | 99.0 % | 93 % | 83 % |

Der Wirkstoff wird in Methylenchlorid gelöst, auf den Träger aufgesprüht und das Lösungsmittel anschließend im Vakuum abgedampft.

| F5. Umhüllungs-Granulate | a) | b) | c) |
|---|---|---|---|
| Wirkstoff gemäß Tabelle 1 | 0.1 % | 5 % | 15 % |
| Polyethylenglykol MG 200 | 1.0 % | 2 % | 3 % |
| Hochdisperse Kieselsäure | 0.9 % | 1 % | 2 % |
| Anorg.Trägermaterial (∅ 0.1 - 1 mm) wie z.B. $CaCO_3$ oder $SiO_2$ | 98.0 % | 92 % | 80 % |

Der fein gemahlene Wirkstoff wird in einem Mischer auf das mit Polyethylenglykol angefeuchtete Trägermaterial gleichmäßig aufgetragen. Auf diese Weise erhält man staubfreie Umhüllungs-Granulate.

| F6. Extruder-Granulate | a) | b) | c) | d) |
|---|---|---|---|---|
| Wirkstoff gemäß Tabelle 1 | 0.1 % | 3% | 5 % | 15 % |
| Na-Ligninsulfonat | 1.5 % | 2 % | 3 % | 4 % |
| Carobxymethylcellulose | 1.4 % | 2 % | 2 % | 2 % |
| Kaolin | 97.0 % | 93 % | 90 % | 79 % |

Der Wirkstoff wird mit den Zusatzstoffen vermischt, vermahlen und mit Wasser angefeuchtet. Dieses Gemisch wird extrudiert und anschließend im Luftstrom getrocknet.

| F7. Stäubemittel | a) | b) | c) |
|---|---|---|---|
| Wirkstoff gemäß Tabelle 1 | 0.1 % | 1 % | 5 % |
| Talkum | 39.9 % | 49% | 35 % |
| Kaolin | 60.0 % | 50 % | 60 % |

Man erhält anwendungsfertige Stäubemittel, indem der Wirkstoff mit den Trägerstoffen vermischt und auf einer geeigneten Mühle vermahlen wird.

| F8. Suspensions-Konzentrate | a) | b) | c) | d) |
|---|---|---|---|---|
| Wirkstoff gemäß Tabelle 1 | 3 % | 10 % | 25 % | 50 % |
| Ethylenglykol | 5 % | 5 % | 5 % | 5 % |
| Nonylphenol-polyglykolether (15 Mol EO) | - | 1 % | 2 % | 1 % |
| Na-Ligninsulfonat | 3 % | 3 % | 4 % | 5 % |
| Carboxymethylcellulose | 1 % | 1 % | 1 % | 1 % |
| 37%ige wäßrige Formaldehyd-Lösung | 0.2 % | 0.2 % | 0.2 % | 0.2 % |
| Silikonöl-Emulsion | 0.8 % | 0.8 % | 0.8 % | 0.8 % |
| Wasser | 87 % | 79 % | 62 % | 38 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Herbizidwirkung vor dem Auflaufen der Pflanzen (pre-emergent)

Kunststofftöpfe werden mit expandiertem Vermiculit (Dichte 0,135 g/cm³, Wasseradsorptionsvermögen 565 g/l) gefüllt. Nach dem Sättigen des nicht adsorptiven Vermiculits mit einer wässrigen Wirkstoffemulsion in deionisiertem Wasser, hergestellt aus einem 25%igen Spritzpulver (Beispiel F3 c)), die die Wirkstoffe in einer Konzentration von 70 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei einer Temperatur von 20°C, einer Beleuchtung von ca.20 kLux und einer relativen Luftfeuchtigkeit von 70 % gehalten. Währen einer Keimphase von 4 bis 5 Tagen werden die Töpfe zur Erhöhung der örtlichen Luftfeuchtigkeit mit lichtdurchlässigem Material abgedeckt und mit deionisiertem Wasser begossen. Nach dem 5. Tag wird dem Giesswasser 0,5 % eines handelsüblichen Flüssigdüngers zugesetzt. Der Versuch wird 12 Tage nach der Aussaat ausgewertet und die Wirkung auf die Versuchspflanzen nach dem folgenden Massstab bewertet:

1 Pflanze hat nicht gekeimt oder ist abgestorben
2-3 sehr starke phytotoxische Wirkung
4-6 mittlere Wirkung
7-8 schwache Wirkung
9 keine Wirkung, die Pflanze wächst wie unbehandelte Kontrollpflanzen.

In diesem Versuch zeigen die geprüften Verbindungen der Tabelle 1 starke Herbizidwirkung.

Beispiele für die gute Herbizidwirkung sind in Tabelle B1 aufgeführt.

Tabelle B1: Pre-emergente Wirkung

| Verb. Nr. | Dosis [µg/Blatt] | Testpflanzen: Nasturtium | Agrostis | Stellaria | Digitaria |
|---|---|---|---|---|---|
| 1.001 | 100 | 3 | 2 | 3 | 3 |
| 1.002 | 100 | 3 | 5 | 3 | 5 |
| 1.004 | 100 | 3 | 2 | 2 | 2 |

Dieselben Resultate werden erhalten, wenn man die Verbindungen der Formel I gemäss den Beispielen F1, F2 und F4 bis F8 formuliert.

Beispiel B2: Post-emergente Herbizid-Wirkung (Kontaktherbizid)

Monokotyle und dikotyle Testpflanzen werden im Gewächshaus in Kunststofftöpfen mit Standarderde angezogen und im 4- bis 6-Blattstadium mit einer wässrigen Suspension der Prüfsubstanzen aus Tabelle 1, hergestellt aus einem 25%igen Spritzpulver (Beispiel F3 b)), besprüht, entsprechend einer Dosierung von 8-500 g Aktivsubstanz pro Hektar (500 l Wasser/ha). Anschliessend werden die Testpflanzen im Gewächshaus unter optimalen Bedingungen weiterkultiviert. Nach ca. 18 Tagen Testdauer wird der Versuch ausgewertet mit einer neunstufigen Notenskala (1 = vollständige Schädigung, 9 = keine Wirkung). Boniturnoten von 1 bis 4 (insbesondere 1 bis 3) bedeuten eine gute bis sehr gute Herbizidwirkung.

In diesem Versuch zeigen die geprüften Verbindungen der Tabelle 1 starke Herbizidwirkung.

Dieselben Resultate werden erhalten, wenn man die Verbindungen der Formel I gemäss den Beispielen F1, F2 und F4 bis F8 formuliert.

**Patentansprüche**

**1.** Verbindungen der Formel I

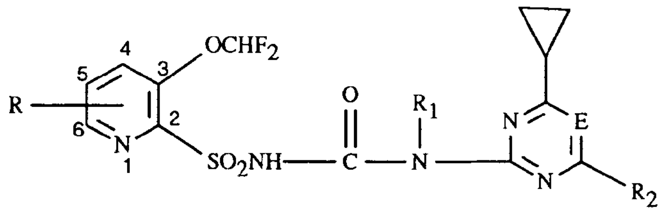

(I),

worin
R und $R_1$ unabhängig voneinander Wasserstoff oder Methyl sind;
$R_2$ Methyl, Methoxy oder Ethoxy; und
E Stickstoff oder die Methingruppe bedeutet, sowie die agrochemisch verträglichen Salze dieser Verbindungen.

2. Verbindungen gemäss Anspruch 1 der Formel I, worin E die Methingruppe bedeutet.

3. Verbindungen gemäss Anspruch 1 der Formel $I_a$

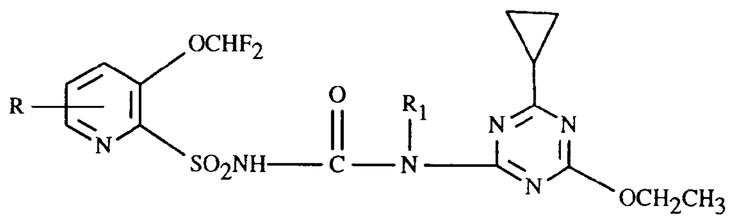

($I_a$),

worin R und $R_1$ die in Anspruch 1 angegebenen Bedeutungen haben.

4. Verbindungen gemäss Anspruch 1 der Formel I, worin R in 6-Position gebunden ist.

5. Verbindung gemäss einem der Ansprüche 1 oder 3 der Formel $I_b$

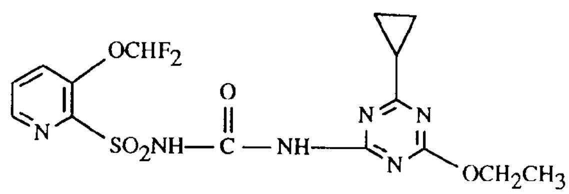

($I_b$).

6. Verfahren zur Herstellung der Verbindungen der Formel I gemäss Anspruch 1, dadurch gekennzeichnet, dass man entweder
a) ein 2-Pyridylsulfonamid der Formel II

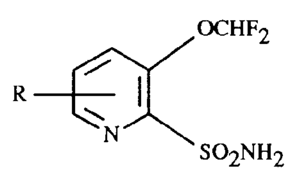

(II),

worin R die in Anspruch 1 angegebene Bedeutung hat, mit einem N-2-Pyrimidinyl- oder N-2-Triazinylcarbamat der Formel III

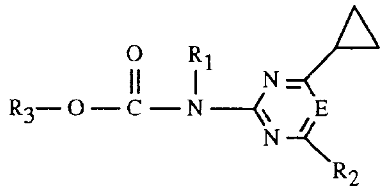

(III),

worin $R_1$, $R_2$ und E die in Anspruch 1 angegebenen Bedeutungen haben und $R_3$ Phenyl oder 4-Tolyl ist, in Gegenwart einer Base umsetzt, oder

b) ein 2-Pyridylsulfonamid der Formel IV

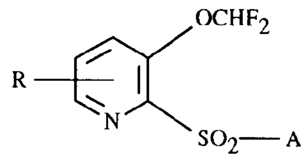

(IV),

worin R die angegebene Bedeutung hat, und A

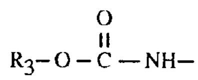

oder O=C=N- bedeutet, wobei $R_3$ die oben angegebene Bedeutung hat, gegebenenfalls in Gegenwart einer Base mit einem 2-Aminopyrimidin oder -triazin der Formel V

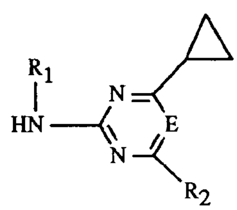

(V),

worin $R_1$, $R_2$ und E die angegebenen Bedeutungen haben, umsetzt, oder

c) ein 2-Pyridylsulfonylchlorid der Formel VI

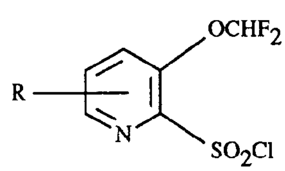

(VI),

worin R die angegebene Bedeutung hat, mit einem Metall-Cyanat der Formel VII

$O{=}C{=}N^{\ominus}M^{\oplus}$ (VII),

worin $M^{\oplus}$ ein Ammonium-, Phosphonium-, Sulfonium- oder ein Alkalimetall-Kation bedeutet, und einem 2-Aminopyrimidin oder -triazin der Formel Va

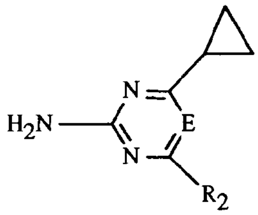

(Va),

worin $R_2$ und E die angegebenen Bedeutungen haben, gegebenenfalls in Gegenwart einer Base in einem inerten, organischen Lösungsmittel umsetzt, oder

d) ein 2-Pyridylsulfonamid der Formel II

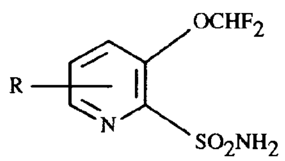

(II),

worin R die angegebene Bedeutung hat, gegebenenfalls in Gegenwart einer Base mit einem 2-Pyrimidinyl- oder 2-Triazinylisocyanat der Formel VIII

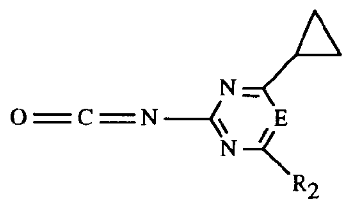

(VIII),

worin $R_2$ und E die angegebenen Bedeutungen haben, umsetzt.

7. Herbizides Mittel, dadurch gekennzeichnet, dass es eine Verbindung der Formel I gemäss Anspruch 1 enthält.

8. Mittel gemäss Anspruch 7, dadurch gekennzeichnet, dass es 0,1% bis 95% einer Verbindung der Formel I gemäss Anspruch 1, 1 bis 99% eines festen oder flüssigen Zusatzstoffes und 0 bis 25%, insbesondere 0,1 bis 25% eines Tensides enthält.

9. Verfahren zur Bekämpfung unerwünschten Pflanzenwachstums, dadurch gekennzeichnet, dass man eine Verbindung der Formel I gemäss Anspruch 1 oder ein diese Verbindung enthaltendes Mittel in einer wirksamen Menge auf die Pflanzen oder deren Lebensraum appliziert.

10. Verfahren gemäss Anspruch 9, dadurch gekennzeichnet, dass man eine Verbindung der Formel I in einer Menge von 0,001 bis 2 kg pro Hektar appliziert.

11. Verfahren gemäss Anspruch 9 zur selektiven pre- oder post-emergenten Bekämpfung von Unkräutern in Nutzpflanzenkulturen, insbesondere Getreide, Baumwolle, Soja, Mais, Reis und bevorzugt Raps.

12. Verwendung einer Verbindung der Formel I gemäss Anspruch 1 zur selektiven pre- oder post-emergenten Bekämpfung von Unkräutern in Nutzpflanzenkulturen, insbesondere Getreide, Baumwolle, Soja, Mais, Reis und bevorzugt Raps.

13. Verwendung eines Mittels gemäss Anspruch 7 zur selektiven pre- oder post-emergenten Bekämpfung von Unkräutern in Nutzpflanzenkulturen, insbesondere Getreide, Baumwolle, Soja, Mais, Reis und bevorzugt Raps.

Europäisches Patentamt

# EUROPÄISCHER RECHERCHENBERICHT

Nummer der Anmeldung

EP 93 81 0525

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y,D | EP-A-0 103 543 (CIBA-GEIGY AG) 21. März 1984 * Seite 28, Verbindungen 137-144 | 1-13 | C07D401/12 A01N47/36 |
| Y,D | EP-A-0 108 708 (CIBA-GEIGY AG) 16. Mai 1984 * Seite 26 - Seite 30; Anspruch 1; Beispiele 15,16 * | 1-13 | |
| A | EP-A-0 206 995 (CIBA-GEIGY AG) 30. Dezember 1986 * Anspruch 1; Tabelle 2 * | 1-13 | |
| P,X | WO-A-9 216 522 (CIBA-GEIGY AG) 1. Oktober 1992 * Tabelle 1, Verbindung1.61 * | 1-13 | |
| | | | RECHERCHIERTE SACHGEBIETE (Int. Cl.5 ) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 07 OKTOBER 1993 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)